# EUROPÄISCHE PATENTANMELDUNG

(11) **EP 1 745 813 A1**
(43) Veröffentlichungstag der Anmeldung: **24.01.2007**
(21) Anmeldenummer: 05015967.2
(22) Anmeldetag: 22.07.2005
(51) Int. Cl.: A61M 5/152, A61M 39/22

(54) **Mechanisch betriebene Flüssigkeitspumpe**

(71) Anmelder: RoweMed AG - Medical 4 Life, 19370 Parchim (DE)
(72) Erfinder: Wex, Roland, 34212 Melsungen (DE); Hansmann, Harald, Prof. Dr.-Ing., 23966 Wismar (DE); Kenzler, Christian, Dipl.-Ing., 16928 Pritzwalk (DE)
(74) Vertreter: Quermann, Helmut

(57) **Zusammenfassung**

Die Erfindung betrifft eine mechanisch betriebene Flüssigkeitspumpe (1), insbesondere für medizinische oder ernährungsphysiologische Flüssigkeiten, mit einem Gehäuse (2) und einem in diesem gehaltenen, aufweitbaren elastischen Element (16) zum Speichern und Abgeben der Flüssigkeit, ferner mit einem verschließbaren Zugang (21) und einem Abgang (50) für die Flüssigkeit vom elastischen Element sowie einer Einrichtung zum weitgehenden Konstanthalten oder Begrenzen des von dem elastischen Element ausgegebenen Flüssigkeits-Volumenstroms.

Erfindungsgemäß sind folgende Merkmale vorgesehen:
- das elastische Element ist als Ballon (16) ausgebildet, der mit einer Öffnung (17a) versehen ist,
- im Gehäuse ist ein Kern (11) gelagert, der durch die Öffnung des Ballons in diesen eingeführt ist,
- es sind Mittel (20) zum Befestigen und Abdichten des Ballons im Bereich seines die Öffnung aufweisenden Endes am Kern oder am Gehäuse vorgesehen,
- in Abstand zu seiner Befestigung liegt der Ballon, in relativ entspanntem Zustand, am Kern an.

## Beschreibung

Die Erfindung betrifft eine mechanisch betriebene Flüssigkeitspumpe, insbesondere für medizinische oder ernährungsphysiologische Flüssigkeiten, sowie Flüssigkeiten im biologischen und Laborbereich, mit einem Gehäuse und einem in diesem gehaltenen, aufweitbaren elastischen Element zum Speichern und Abgeben der Flüssigkeit, ferner mit einem verschließbaren Zugang für die Flüssigkeit zum elastischen Element, einem Abgang für die Flüssigkeit vom elastischen Element und einer Einrichtung zum weitgehenden Konstanthalten und/oder Begrenzen des von dem elastischen Element ausgegebenen Flüssigkeits-Volumenstroms.

Im Zusammenhang mit der Förderung medizinischer, ernährungsphysiologischer oder biologischer Flüssigkeiten oder Flüssigkeiten im Laborbereich werden die unterschiedlichsten Typen von Flüssigkeitspumpen verwendet. Es sind beispielsweise elektroenergetisch, elektrochemisch, gasförmig, mechanisch, elektromechanisch und mechanisch-physikalisch betriebene Pumpen bekannt. Viele dieser Pumpen können in aller Regel nur nach einer längeren Anlaufzeit eingesetzt werden und bieten dem Anwender und Verbraucher oftmals nur eine unzureichende Dosiergenauigkeit. Die bekannten Pumpensysteme sind im Übrigen recht kostenaufwendig und unter ökologischem Gesichtspunkt wenig Vorteilhaft. Sie können oftmals vom Anwender nicht tragbar benutzt werden.

Mechanisch betriebene Flüssigkeitspumpen zeichnen sich durch einen Antrieb aus, der in aller Regel recht einfach gestaltet sein kann. So sind die unterschiedliche Ausführungsformen bekannt geworden, bei denen aufweitbare elastische Elemente zum Speichern und Abgeben der Flüssigkeit Verwendung finden.

Eine mechanisch betriebene Flüssigkeitspumpe, insbesondere für medizinische oder ernährungsphysiologische Flüssigkeiten ist aus der EP 0 944 405 B1 bekannt. Bei dieser ist das aufweitbare elastische Element als Schlauch ausgebildet. Dieser ist innerhalb eines Rohres angeordnet, das im Bereich eines Endes mittels einer stationären Endkappe verschlossen ist. In dieser Endkappe ist der Schlauch mit seinem einen offenen Ende abgedichtet gelagert. In das Schlauchende führt eine Leitung, die die Endkappe durchsetzt. Abgewandt dieser Endkappe ist im Rohr eine weitere Endkappe verschieblich gelagert, die den Schlauch im Bereich seines anderen, offenen Endes aufnimmt. Dort ist der Schlauch entsprechend gelagert, somit abgedichtet in dieser verschieblichen Endkappe, und es ist eine Leitung durch diese Endkappe hindurch gesteckt und in den Schlauch hineingeführt. Der der beweglichen Endkappe zugeordneten Leitung ist ein Ventil zugeordnet, durch das die Flüssigkeit in den Schlauch einströmen kann, wobei der Schlauch gedehnt wird, bis er an der Innenwandung des Rohres anliegt. Auf Grund der Dehnung des Schlauches wird auch die bewegliche Endkappe von der stationären Endkappe weg bewegt. Unter der elastischen Rückstellkraft des Schlauches, die radial nach innen wirkt, wird die Flüssigkeit durch die der stationalen Endkappe zugeordnete Leitung zum Patienten abgeführt.

Eine solche Gestaltung der mechanisch betriebenen Flüssigkeitspumpe ist aufwendig, weil ein elastisches Element innerhalb eines Schlauches Verwendung findet, der somit zwei diametrale Öffnungen aufweist, denen separate Leitungen - eine mit der Funktion des Zugangs, die andere mit der Funktion des Abgangs - zugeordnet sind. Abgesehen hiervon kann mittels des Schlauches die Flüssigkeit nicht vollständig aus dem Schlauch abgegeben und damit dem Patienten zugeführt werden, weil mit zunehmender Annäherung der Schlauchwandungen in Folge Abgabe der Flüssigkeit an den Patienten die elastischen Rückstellkräfte des Schlauches nachlassen. Schließlich dehnt sich der Schlauch radial gleichmäßig nach außen aus, bis er an der Innenwand des Rohres anliegt. Auf Grund der Vorgabe der rotationssymmetrischen Gestaltung des Rohres ist eine ungünstige Abmessung der Pumpe in Breiten-Tiefen-Erstreckung vorgegeben. Es kann somit keine flachbauende Pumpe auf Grund der Ausbildung des elastischen Elements in Art eines Schlauches dargestellt werden. Die Abdichtung des Schlauches ist aufwendig, da sie an zwei Stellen zu erfolgen hat.

Eine ähnlich gestalte, mechanisch betriebene Flüssigkeitspumpe ist in der EP 0 424 994 B1 beschrieben.

Es sind mechanisch betriebene Flüssigkeitspumpen, insbesondere für medizinische oder ernährungsphysiologische Flüssigkeiten bekannt geworden, bei denen ein Beutel zur Aufnahme der Flüssigkeit Verwendung findet, wobei eine mit dem Beutel verbundene Leitung zur Ausgabe der im Beutel befindlichen Flüssigkeit aus dem Beutel dient. Der Beutel wirkt mit einer Einrichtung zum Ausüben einer Druckkraft auf den Beutel zwecks Ausgabe der Flüssigkeit aus diesem zusammen. Die Einrichtung erweist ein elastisches Element zum Einwirken auf eine Beutelseite sowie ein weiteres Element zum Einwirken auf die gegenüberliegende Beutelseite auf. Eine solche Flüssigkeitspumpe ist in der DE 199 28 131 A1 beschrieben. Ähnliche mechanisch betriebene Flüssigkeitspumpen, die auf diesem Wirkprinzip beruhen, sind in der DE 100 63 975 A1 und DE 100 58 121 A1 beschrieben.

In der DE-OS 27 01 100 ist eine Pumpe beschrieben, die insbesondere auf dem Gebiet der Chromatographie Verwendung findet. Bei dieser wird eine Flüssigkeit, zum Beispiel ein Eluiermittel, durch Zufuhr eines Druckgases ausgetrieben. Die Pumpe weist einen flexiblen Behälter in Form einer schlauchförmigen Membran auf, der an einem Ende mit einer Schraubklemme abgedichtet ist. Den Schlauch umgibt abgedichtet ein Rohr, womit ein Raum zwischen diesem und dem Schlauch gebildet ist. Bei der chromatographischen Anwendung drückt in diesen Raum eingeführter Stickstoff den Schlauch zusammen, wobei das gesamte Eluiermittel oder ein Teil davon ausgestoßen und durch eine chromatographische Säule gedrückt wird.

Aufgabe der Erfindung ist es, eine mechanisch betriebene Flüssigkeitspumpe der Eingangs genannten Art so weiterzubilden, dass bei einfacher Gestaltung des Antriebs der Pumpe eine vollständige Ausgabe der Flüssigkeit gewährleistet ist.

Gelöst wird die Aufgabe bei einer Flüssigkeitspumpe der Eingangs genannten Art durch folgende Merkmale:
- das elastische Element ist als Ballon ausgebildet, der mit einer Öffnung versehen ist,
- im Gehäuse ist ein Kern gelagert, der durch die Öffnung des Ballons in diesen eingeführt ist,
- es sind Mittel zum Befestigen und Abdichten des Ballons im Bereich seines die Öffnung aufweisenden Endes am Kern oder am Gehäuse vorgesehen,
- in Abstand von seiner Befestigung liegt der Ballon, bei relativ entspanntem Zustand, am Kern an.

Der Antrieb der erfindungsgemäßen mechanisch betriebenen Flüssigkeitspumpe zeichnet sich bereits dadurch in besonderer Art und Weise aus, dass das elastische Element als Ballon ausgebildet ist. Es handelt sich somit um ein elastisches Element, das mit einer einzigen Öffnung versehen ist. Demzufolge dient diese Öffnung sowohl als Zugang für die Flüssigkeit als auch als Abgang für die Flüssigkeit. Eine weitere Besonderheit ist darin zu sehen, dass mit dem Ballon ein Kern zusammenwirkt, der durch die Öffnung des Ballons in diesen eingeführt ist. Der Ballon liegt in relativ entspanntem Zustand am Kern an. Hierunter ist zu verstehen, dass der Ballon durchaus mit einer gewissen Vorspannung am Kern anliegen kann, da die vollständige Entleerung des Ballons anzustreben ist. Befestigt und abgedichtet wird der Ballon ausschließlich im Bereich seines die Öffnung aufweisenden Endes. Dies erfolgt vorzugsweise unmittelbar am Kern. Es ist genauso denkbar, die Befestigung und Abdichtung des Ballons am Gehäuse vorzusehen.

Der Ballon kann grundsätzlich aus jedem Material gebildet sein, das die notwendige Elastizität zum Speichern und Abgeben der Flüssigkeit aufweist. Als bevorzugtes Material wird Silikon angesehen. Es ist insbesondere an ein Fassungsvermögen des Ballons von 10 ml bis 150 ml gedacht.

Bei der Flüssigkeitspumpe wird es als besonders wichtig angesehen, dass diese relativ flach ausgebildet ist, da sie in aller Regel von einem Patienten unmittelbar am Körper getragen wird. Eine Flüssigkeitspumpe die einen kreisförmigen Durchmesser aufweist, ist unter diesem Aspekt wenig vorteilhaft.

Um eine Ausdehnung des Ballons vorzugsweise in Breitenerstreckung und weniger in seiner Höhe zu erreichen, sieht eine Weiterbildung der Erfindung vor, dass der Ballon in einer ersten Erstreckungsrichtung senkrecht zur Längsachse des Kerns relativ dicke Wandungsabschnitte und in einer zweiten Erstreckungsrichtung senkrecht zur Längsachse des Kerns und senkrecht zur ersten Erstreckungsrichtung relativ dünne Wandungsabschnitte aufweist. Das bedeutet, dass sich der Ballon stärker im Bereich der dünnen Wandungsabschnitte verformt, womit sich beim Dehnen des Elements eine im Wesentlichen an die Form einer Elypse angenäherte Querschnittsgestaltung ergibt. Der Ballon ist insbesondere durch Spritzgießen hergestellt.

Gemäß einer bevorzugten Weiterbildung der Erfindung ist vorgesehen, dass mit dem Gehäuse eine Haube verbindbar ist, insbesondere eine im Querschnitt, somit quer zur Längsachse des Kerns gesehen, nierenförmig gestaltete Haube verbindbar ist. Innerhalb dieser Haube ist der Ballon angeordnet. Durch die nierenförmige Gestaltung der Haube kann diese sich günstig der Körperkontur des die Pumpe Tragenden im Bereich dessen Hüfte anschmiegen. Der mit unterschiedlichen dicken Wandungsabschnitten gestaltete Ballon folgt beim Ausdehnen ungefähr der Innenkontur der dünnwandig gestalteten nierenförmigen Haube. Bei hinreichendem Befüllen des Ballons mit Flüssigkeit kann sich dieser an die Innenwandung der Haube anlegen. Das Breiten-/Höhenverhältnis der Haube ist relativ groß, es beträgt vorzugsweise 1,5:1 bis 3:1, insbesondere ungefähr 2:1. Die Haube ist vorzugsweise unlösbar mit dem Gehäuse verclipsbar.

Bei einer bevorzugten Gestaltung ist der Kern rotationssymmetrisch ausgebildet, insbesondere zylindrisch. Er weist somit, abgesehen von einer eventuellen Verdickung im Verbindungsbereich zum Gehäuse, einen gleichbleibenden Durchmesser auf. Entsprechend weist der Ballon in entspanntem Zustand, somit dann, wenn er unabhängig vom Kern positioniert ist, gleichfalls Zylinderform auf, wobei der Durchmesser des Zylinders geringer ist als der Durchmesser des Kerns, bezogen auf den Bereich des Kerns, mit dem der Ballon in relativ entspanntem Zustand zur Anlage gelangt. Es wird als besonders vorteilhaft angesehen, wenn der Ballon vollständig an den Kern anlegbar ist, sodass sich dann keine Flüssigkeit mehr zwischen Ballon und Kern befindet.

Zum Einführen der Flüssigkeit in den Ballon und dem Ausgeben der Flüssigkeit aus dem Ballon kann der Kern auf unterschiedliche Art und Weise gestaltet sein. Vorzugsweise durchsetzt den Kern ein Kanal, der Bestandteil des Zugangs und des Abgangs bildet. Der Kanal durchsetzt den Kern insbesondere in dessen Längsachse und in dessen radialer Richtung. Ausgehend von dem in der Längsmittelachse verlaufenden Bereich des Kanals kann Flüssigkeit in die radialen Abschnitte dieses Kanals gelangen, womit über diese axial und radial verlaufenden Kanalabschnitte eine Verbindung zum Inneren des Ballons sichergestellt ist. Das Längen-Durchmesserverhältnis des Kerns beträgt 3:1 bis 8:1, insbesondere 5:1 bis 6:1.

Die Mittel zum Befestigen und Abdichten des Ballons sind vorzugsweise als Hülse ausgebildet, die, bei auf den Kern aufgezogenem Ballon, diesen zwischen der Hülse und den Kern klemmt.

Weitere Merkmale der Erfindung sind in den Unteransprüchen, der Beschreibung der Figuren sowie den Figuren selbst dargestellt, wobei bemerkt wird, dass alle Einzelmerkmale und alle Kombinationen von Einzelmerkmalen erfindungswesentlich sind.

In den Figuren ist die Erfindung anhand einer Ausführungsform beispielsweise dargestellt, ohne hierauf beschränkt zu sein. Es zeigt:
- Figur 1: eine Explosionsdarstellung der erfindungsgemäßen mechanisch betriebenen Flüssigkeitspumpe,
- Figur 2: einen vertikalen Längsmittelschnitt durch die in Figur 1 gezeigte Pumpe, bei am Kern anliegendem Ballon,
- Figur 3: ein Schnitt gemäß Figur 2, bei mit Flüssigkeit gefülltem Ballon,
- Figur 4: einen vertikalen Längsschnitt durch die in Figur 1 gezeigte Pumpe, in Abstand zur Längsmittelachse der Pumpe, im Bereich eines Ventils der Pumpe geschnitten,
- Figur 5: einen Schnitt, quer durch die in Figur 1 veranschaulichte Pumpe geschnitten, im Bereich des Ventils,
- Figur 6: einen horizontalen Längsmittelschnitt durch die in Figur 1 gezeigte Pumpe, bei am Kern anliegenden Ballon,
- Figur 7: einen Schnitt gemäß Figur 6, bei mit Flüssigkeit gefülltem Ballon,
- Figur 8: einen Schnitt durch die Pumpe, quer geschnitten im Bereich der Lagerung des Kernes,
- Figur 9: einen Schnitt durch die Pumpe, quer geschnitten im Bereich des nicht gelagerten Abschnitts des Kernes und des mit Flüssigkeit gefüllten Ballons,
- Figur 10: eine vergrößerte Schnittdarstellung von Kern, Ballon und Klemmring zum Verbinden von Ballon und Kern, bei am Kern anliegenden Ballon,
- Figur 11: einen Schnitt, quer zur Längserstreckung des Kerns geschnitten, durch den Kern und den Ballon, bei am Kern anliegenden Ballon,
- Figur 12: eine Schnittdarstellung gemäß Figur 11 für eine modifizierte Querschnittsgestaltung des Ballons,
- Figur 13: eine vergrößerte Schnittdarstellung des in Figur 4 gezeigten Ventils, und
- Figur 14: ein Schaubild zur Verdeutlichung des Wirkprinzips der mechanisch betriebenen Flüssigkeitspumpe mit Angabe physikalischer Größen.

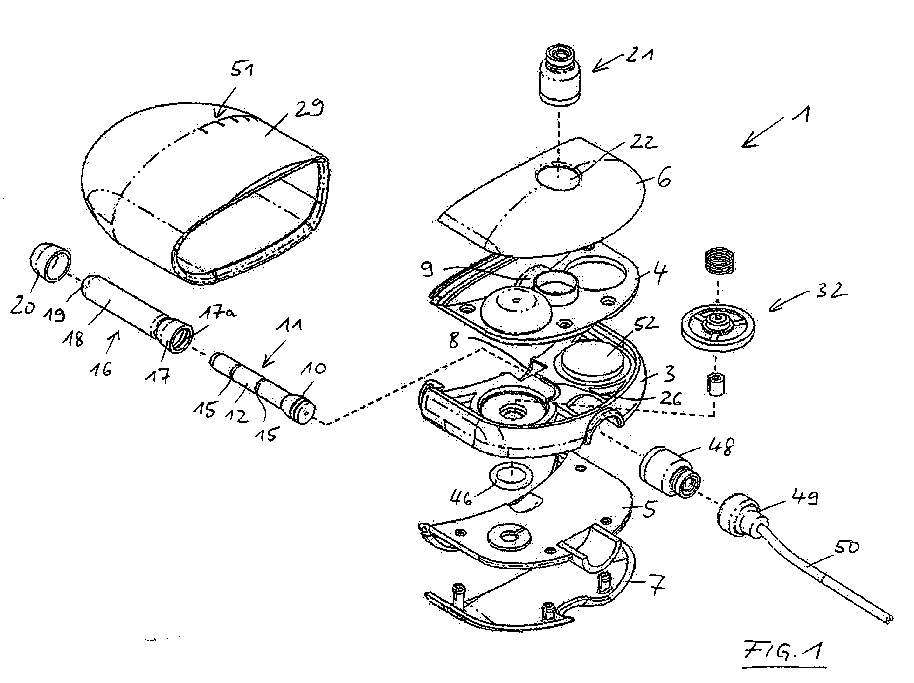

Die in Figur 1 veranschaulichte mechanisch betriebene Flüssigkeitspumpe 1 dient insbesondere der Applikation von medizinischen oder ernährungsphysiologischen Flüssigkeiten, beispielsweise der Applikation eines flüssigen Medikaments.

Die Pumpe 1 weist ein mehrteiliges Gehäuse 2 auf, das durch ein Mittelteil 3, mit diesem zusammenwirkenden Oberteil 4 und Unterteil 5, eine mit dem Oberteil zusammenwirkende Oberschale 6 und eine mit dem Unterteil 5 zusammenwirkende Unterschale 7 gebildet ist.

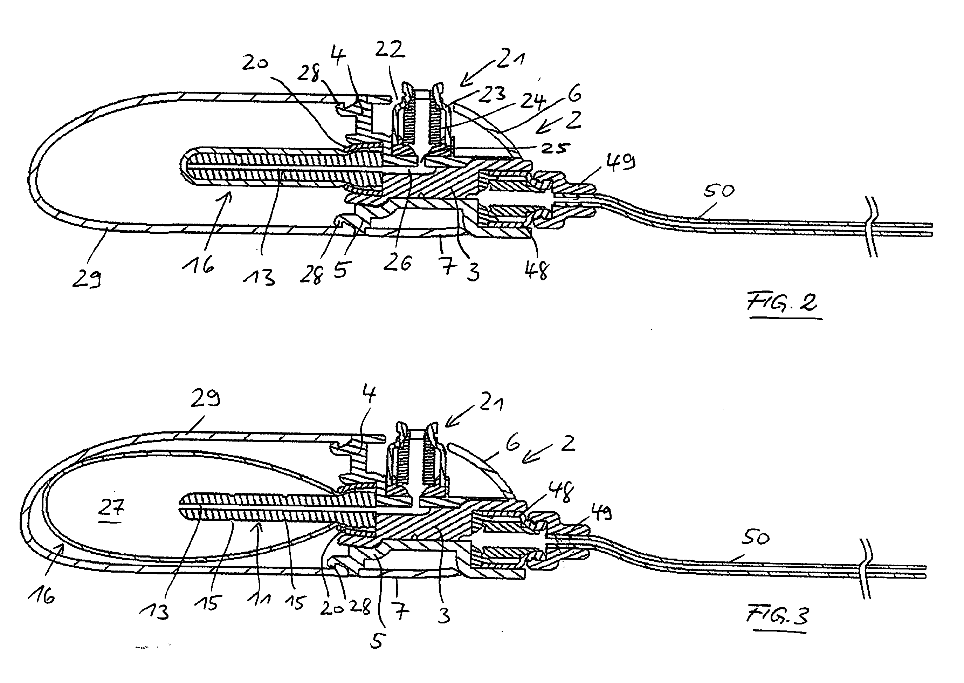

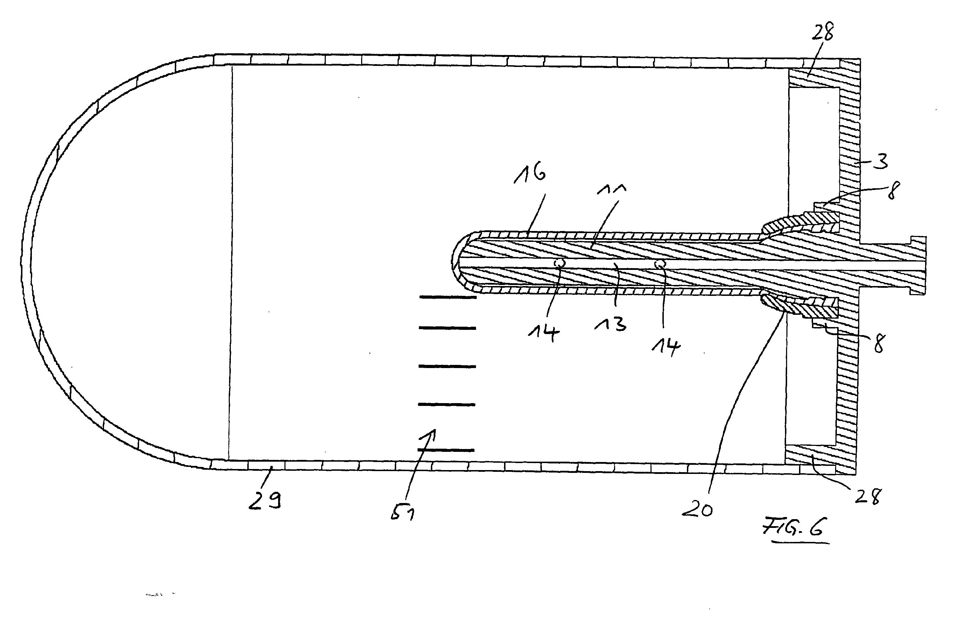

Das Mittelteil 3 ist auf seiner Oberseite mit einer zum freien Rand des Mittelteils 3 offenen, im Querschnitt halbkreisförmigen Ausnehmung 8 und das Oberteil 4 im entsprechenden Randbereich auf seiner Unterseite mit einer entsprechenden halbkreisförmigen Ausnehmung 9 versehen. Bei mit dem Mittelteil 3 verbundenem Oberteil 4 bilden die beiden Ausnehmungen 8 und 9 einen kreisförmigen Querschnitt zur Aufnahme eines konusförmig erweiterten Endbereichs 10 eines Kernes 11. Dieser weist, abgesehen von dessen Endbereich 10, einen konstanten Außendurchmesser auf. Dieser zylindrische Abschnitt des Kerns 11 ist mit der Bezugsziffer 12 bezeichnet. Den Kern 11 durchsetzt in dessen Längsmittelachse ein Kanal 13 (siehe Figur 2) von dem mehrere sich radial durch den Kern 11 erstreckende Kanäle 14 im Bereich des Abschnitts 12 abzweigen (Figur 6). Im Bereich des äußeren Umfangs des Kerns 10 münden die radialen Kanäle 14 in umlaufende Nuten 15 des Kerns 11.

Mit dem Kern 11 wirkt ein elastisches Element zusammen, dass als aus Silikon bestehender Ballon 16 ausgebildet ist. Dieser ist im Spritzgussverfahren hergestellt. Der Ballon weist entsprechend dem Endbereich 10 des Kerns 11 einen sich konisch erweiterten Endbereich 17 mit Öffnung 17a und einen der äußeren Form des Abschnitts 12 des Kerns 11 entsprechenden Abschnitt 18 auf, der in den, Ballon bedingt, geschlossenen Endbereich 19 mündet, der dem Endbereich 17 abgewandt ist.

Die Abmessungen von Kern 11 und Ballon 16 sind so bemessen, dass, wie es der Figur 2 zu entnehmen ist, der auf den Kern 11 aufgesteckte Ballon vollständig am Kern 11 anliegt, somit der Endbereich 17 des Ballons den Endbereich 10 des Kerns kontaktiert und der Abschnitt 18 des Ballons 16 den Abschnitt 12 des Kerns 11 kontaktiert, schließlich der Endbereich 19 des Ballons 16 am stirnseitigen freien Ende des Kerns 11 anliegt. Die Abmessungen des Ballons 16 bezüglich des Kerns 11 sind hierbei so gewählt, dass der Ballon 16 mit relativ geringer Vorspannung, somit in relativ entspanntem Zustand am Kern 11 anliegt.

Zum Befestigen des Ballons 16 im Bereich seines Endbereichs 17 am Kern 11 im Bereich dessen Endbereichs 10 ist ein Klemmring 20 vorgesehen, der außen auf den Ballon 16 im Bereich dessen Endbereichs 17 aufgesteckt wird. Das so geschaffene Gebilde wird mit dem Klemmring 20 in die Ausnehmung 8 des Mittelteils 3 eingelegt, anschließend das Oberteil 4 mit dem Mittelteil 3 verbunden, womit der Klemmring 20 und damit der Kern 11 sowie der Ballon 16 fest in den Ausnehmungen 8 und 9 von Mittelteil 3 und Oberteil 4 gehalten sind. Die Ausnehmungen 8 und 9 weisen eine sich vom jeweiligen freien Rand des Mittelteils 3 bzw. Oberteil 4 weg erweiternde konische Aufnahme für den Klemmring 20 auf, um einen sicheren Halt des Klemmrings 20 zu gewährleisten.

Das Mittelteil 3, das Oberteil 4 und das Unterteil 5 dienen der Aufnahme weiterer Funktionselemente der Pumpe 1:

Mit dem Oberteil 4 ist ein LuerLock-Ventil 21 verbunden, dass eine Öffnung 22 im Oberteil 4 durchsetzt und, wie es der nachfolgend Bezug genommenen Beschreibung der Figur 2 zu entnehmen ist, ein LuerLock-Ventilgehäuse 23 und einen LuerLock-Ventilkern 24 besitzt. Über einen Kanal 25 steht das LuerLock-Ventil 21 mit einem Kanal 26 in Verbindung, der zwischen dem Oberteil 4 und dem Mittelteil 3 gebildet ist und der mit dem den Kern 11 durchsetzenden Kanal 13 in Verbindung steht.

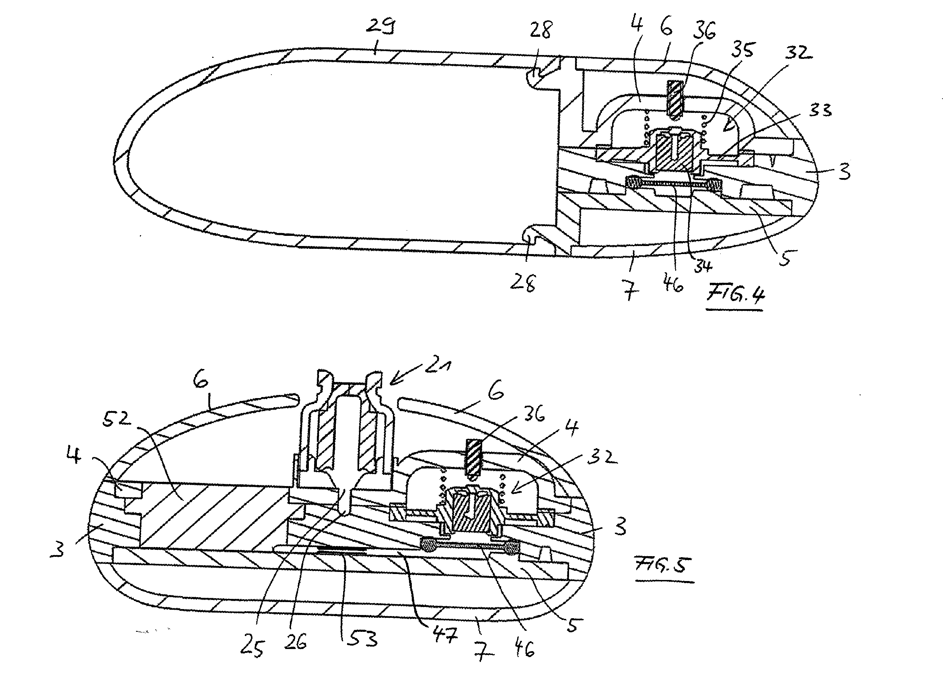

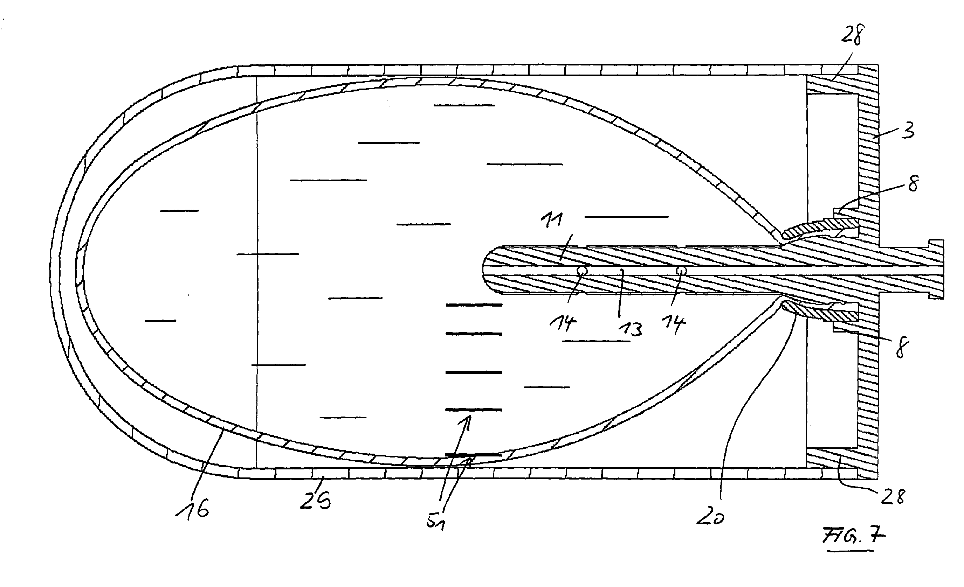

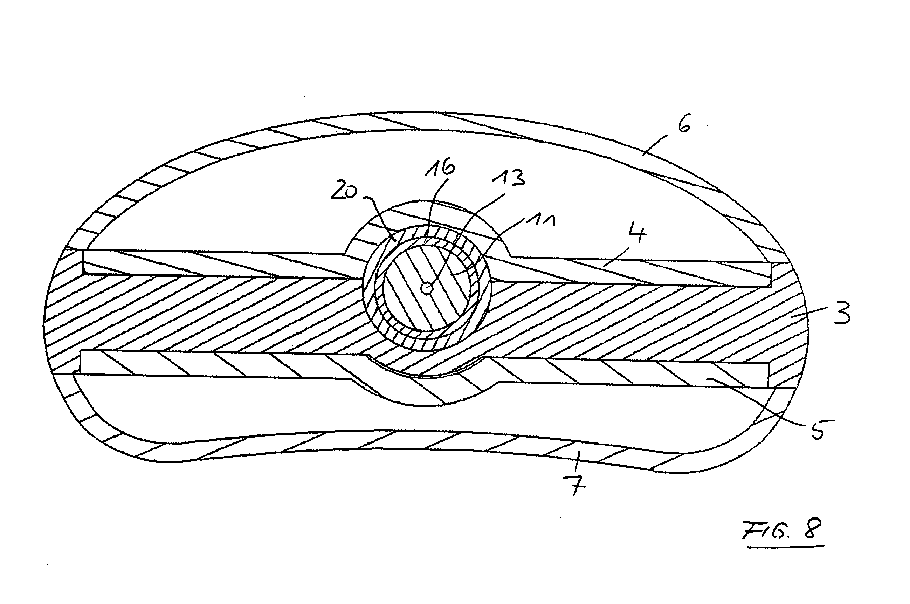

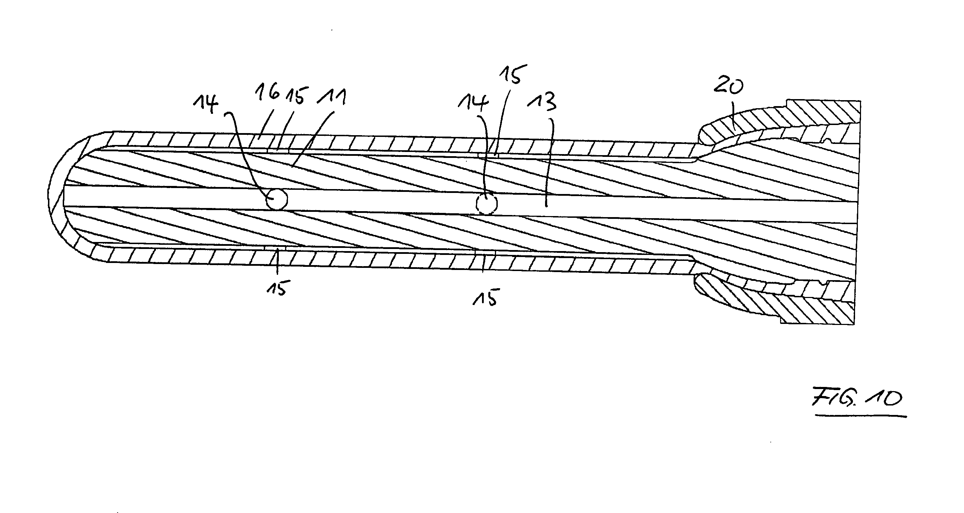

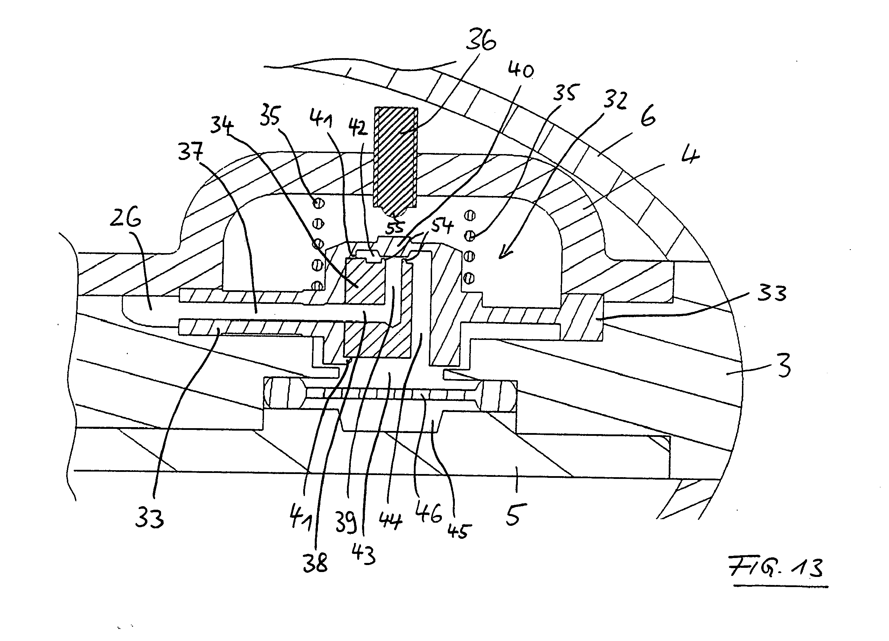

Durch das LuerLock-Ventil 31 und die Kanäle 25, 26 und 13 wird die Pumpe mit Flüssigkeit befüllt. Ausgehend von dem in Figur 2 veranschaulichten unbefüllten Zustand dehnt sich mit zunehmender Zugabe von Flüssigkeit der Ballon 16 in demjenigen Bereich, der nicht durch den Klemmring 20 geklemmt ist und nimmt die in Figur 3 veranschaulichte Endform bei vollständiger Füllung an. Der Raum, der von der Flüssigkeit eingenommen wird, ist dort mit der Bezugsziffer 27 bezeichnet. Den Figuren 2, 3 und 6 bis 12 ist zu entnehmen, dass sich der Ballon 16, ausgehend von seinem am Kern 11 anliegenden Ausgangszustand beim Befüllen mit Flüssigkeit seine Form sowohl in Längsrichtung des Kerns als auch in dessen Querrichtungen verändert, d. h. in einer ersten Querrichtung und einer senkrechten hierzu gesehen zweiten Querrichtung.

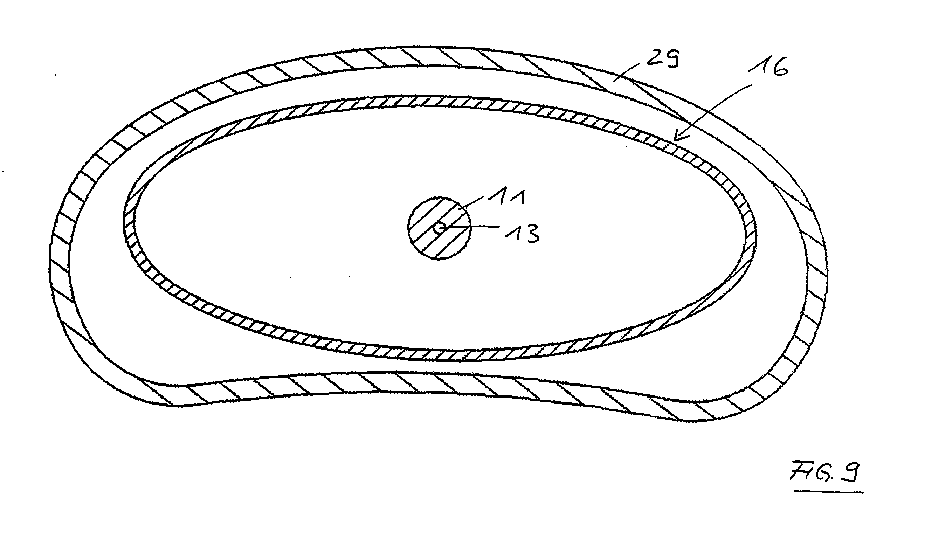

Das Oberteil 4 und das Unterteil 5 sind mit Rastvorsprüngen 28 versehen, die der Aufnahme einer im Querschnitt annähernd nierenförmig gestalteten Haube 29 dienen. Diese Haube besitzt, wie es anschaulich der Figur 9 zu entnehmen ist, eine Erstreckung in Breitenrichtung, die wesentlich größer ist als die in Höhenrichtung. Das Breiten-/Höhenverhältnis beträgt beispielsweise 2:1. Das

Längen-/Höhenverhältnis der Haube 29 ist, wie es beispielsweise der Figur 2 zu entnehmen ist, ungefähr 2,5:1. Die Haube 29 ist vorzugsweise unlösbar mit dem Gehäuse 2 verclipst. Bei vollständig mit Flüssigkeit befülltem Ballon 16 nimmt dieser soviel wie möglich von dem Innenraum der Haube 29 ein.

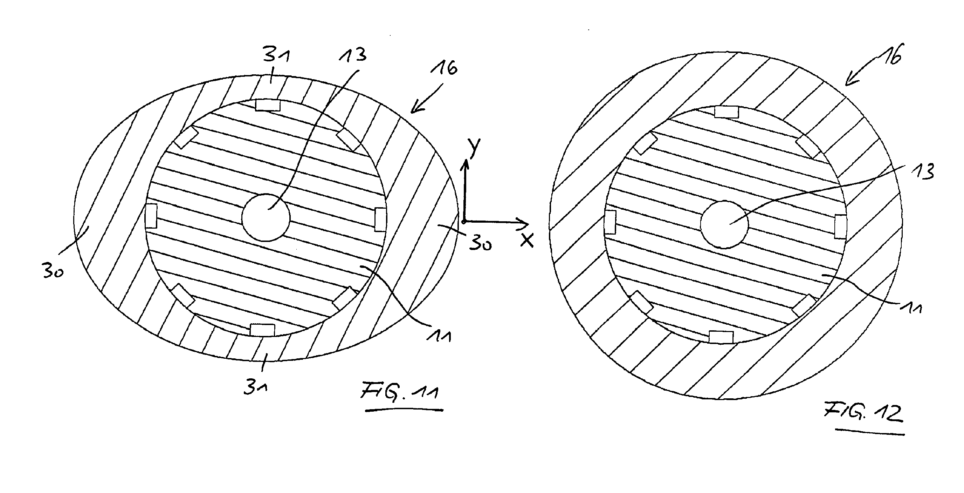

Erreicht wird dies dadurch, dass, wie es der Darstellung der Figur 11 für den am Kern 11 anliegenden Ballon 16 zu entnehmen ist, der Ballon 16 in einer ersten Erstreckungsrichtung X senkrecht zur Längsachse des Kerns 11 relativ dicke Wandungsabschnitte 30 und in einer zweiten Erstreckungsrichtung Y senkrecht zur Längsachse des Kerns 11 und senkrecht zur ersten Erstreckungsrichtung X relativ dünne Wandungsabschnitte 31 aufweist. Demzufolge hat der Ballon 16 beim Einbringen von Flüssigkeit in dessen Raum 27 das Bestreben sich bevorzugt in Erstreckungsrichtung X auszudehnen, womit die ausgedehnte ovale Querschnittsform, wie sie in der Darstellung der Figur 9 veranschaulicht ist, sich ergibt. Insgesamt stellt sich die Pumpe 1 als flaches, günstig am Körper zu tragendes Funktionsteil dar, wobei der Ballon 16 in mit Flüssigkeit gefülltem Zustand gleichfalls eine flache Form einnimmt, die der äußeren Kontur der Pumpe 1 angepasst ist.

Die Kanäle 26 und 13 dienen nicht nur dem Zuführen der Flüssigkeit vom LuerLock-Ventil 21 in den Ballon 16, sondern auch dem Abführen der Flüssigkeit aus dem Inneren des Ballons 16 zum Patienten. So ist der Kanal 26 über die Zugangsstelle des Kanals 25 hinaus verlängert zu einem im Mittelteil 3 und im Oberteil 4 gelagerten Ventil 32 zum Begrenzen des von dem Ballon 16 ausgegebenen Flüssigkeits-Volumenstroms. Dieses Ventil 32 ist durch eine randseitig zwischen Mittelteil 3 und Oberteil 4 gehaltene elastische Ventilmembran 33, einen mit dieser zusammenwirkenden Ventilkern 34, eine sich an der Ventilmembran 33 und dem Oberteil 4 abstützende Druckfeder 35 und eine in einem Gewinde des Oberteils 4 gelagerte Stellschraube 36, die in Wirkverbindung mit der Ventilmembran 33 bringbar ist, gebildet.

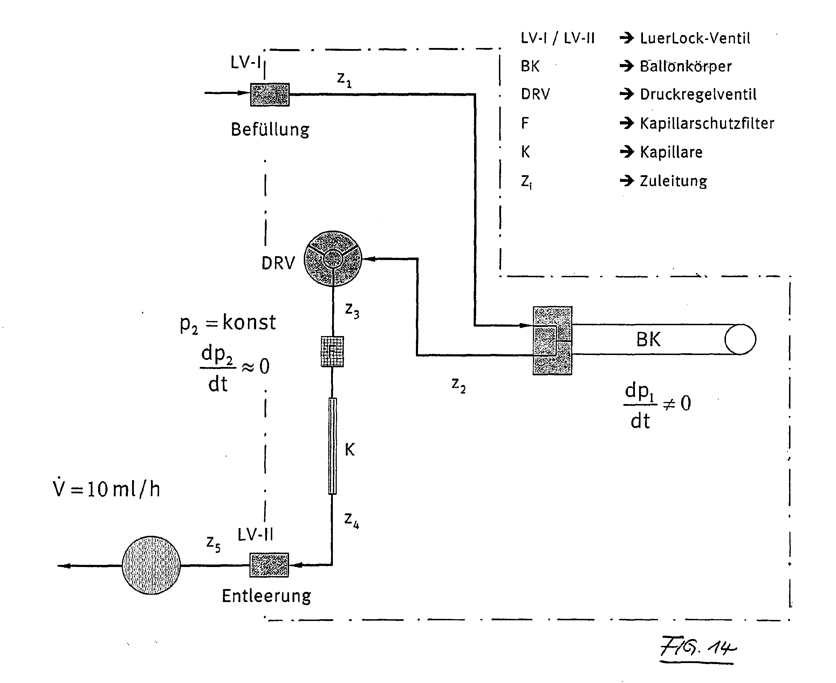

Wie im Detail der Darstellung der Figur 13 zu entnehmen ist, mündet der Kanal 26 in einen radial verlaufenden Kanal 37 der Ventilmembran 33 und von dort in einen radialen Kanal 38 des Ventilkörpers 34, der in einen axialen Kanal 39 des Ventilkerns 34 mündet. Dieser Kanal 39 ist im Bereich seines, einem verstärkten Abschnitt 40 der Ventilmembran 33 zugewandten Endes offen ausgebildet. Auf der dem Kanal 39 abgewandten Seite des Abschnitts 40, dem die Funktion eines Verschlusselements zukommt, ist ein Anschlag, der als Stellschraube 36 ausgebildet ist, angeordnet. Grundsätzlich könnte dieser Anschlag auch stationär sein. Zwischen den Vorsprüngen 41 der Ventilmembran 33 ist der Ventilkern 34 axial unverschieblich bezüglich der Ventilmembran 33 gehalten und im Übrigen auch bezüglich dieser nicht drehbar.

Das Ventil 32 dient dem Sperren des Volumenstromes bei Anliegen eines zu hohen Drucks. Im Ventil sind zwei getrennte Kammern 42 und 43 gebildet, die miteinander über einen den Ventilkern 34 durchsetzenden, parallel zum Kanal 40 angeordneten Kanal 44 verbunden sind. Dabei dient die Kammer 42, welche in Strömungsrichtung zum Zugang, demnach zum Kanal 26 liegt, als Sperrkammer. Die Kammer 43 liegt in Strömungsrichtung zum Abgang 45. Zum Filtern der durch das Ventil 32 ausgegebenen Flüssigkeit ist ein Filter 46 vorgesehen, der randseitig zwischen dem Mittelteil 3 und dem Unterteil 5 geklemmt ist. Ausgehend von der Kammer 43 und dem Abgang 45 gelangt die Flüssigkeit zu einem mit dem Abgang 45 in Fließverbindung stehenden Kanal 47 (Figur 5) und von dort zu einem zwischen dem Mittelteil 3 und dem Unterteil 5 gehaltenen LuerLock-Anschluss 48. Mit diesem ist ein Luer-Lock-Verbinder 49 verbindbar, der mit einem Schlauch 50, der zum Patienten führt, versehen ist.

Wie der Darstellung der Figur 5 zu entnehmen ist, ist in den Kanal 47 eine Glaskapillare 53 eingesetzt. Diese stellt einen Durchflussbegrenzer dar, der in der Lage ist, den Volumenstrom, der durch den Kanal 47 aus der Pumpe austritt, zu begrenzen, weil der Durchflussbegrenzer einen kleineren Querschnitt aufweist, als der im Zugang liegende Kanal 37. Durch die Wahl verschiedener Durchflussbegrenzer ist die Einstellung verschiedener konstanter Flowraten möglich, solange der Eingangs des Eintritts anliegende Druck einen bestimmten Wert nicht unterschreitet. Grundsätzlich ist vorgesehen, dass der Durchflussquerschnitt des Zugangs größer ist als der Durchflussquerschnitt des Abgangs. Selbstverständlich kann der Durchflussbegrenzer anders ausgeführt sein, als in Art einer Glaskapillare. Es ist durchaus denkbar, hinter dem Ventil im Abgang der Pumpe, beispielsweise einen Mäander-Chip, der den Durchfluss begrenzt, vorzusehen.

Auf Grund der angegebenen Durchmesser der Kanäle, die den Raum 27 des Ballons mit dem Ventil 32 verbinden und des Durchmessers der hinter dem Ventil 32 angeordneten Kanäle mit dem Durchflussbegrenzer 53, ist der Widerstand, den der Kanal 47 mit dem Durchflussbegrenzer 53 dem Auströmen der Flüssigkeit aus dem Gehäuse 2 entgegensetzt, größer als der Widerstand, der der Flüssigkeit beim Einströmen in das Ventil 32 entgegen gesetzt wird.

In einer Ausgangssituation befindet sich die Ventilmembran 33 in der in Figur 13 gezeigten Position, in der die Ventilmembran 33, ohne dass es einer Einwirkung der Druckfeder 35 bedürfte, weitgehend am Mittelteil 3 anliegt. Auf Grund der Positionierung des Ventilkerns 34 bezüglich des Abschnitts 40 der Ventilmembran 33 ist ein geringer Spalt zwischen dem Abschnitt 40 und einem umlaufenden, somit ringförmigen Vorsprung 54 des Ventilkerns 34 gegeben. Dieser Vorsprung 54 umschließt den Kanal 43. Demzufolge strömt Flüssigkeit über den Kanal 13 des Kerns 11 und den anschließenden, gehäuseseitigen Kanal 26 in den Kanal 37 der Ventilmembran 33 und von dort in die Kanäle 38 und 39 des Ventilkerns 34. Vom Kanal 39 des Ventilkerns 34 strömt die Flüssigkeit durch den zwischen dem Vorsprung 54 und dem Abschnitt 40 der Ventilmembran 33 gebildeten Spalt in die dort befindliche Kammer 42, und von der Kammer 42 über den Kanal 44 zwischen Ventilmembran 33 und Ventilkern 34 zur Kammer 43, passiert den Filter 46 und gelangt über den Abgang 45 zum Kanal 47 mit dem Durchflussbegrenzer 53. Stellt sich im Einlass, somit auch im Kanal 39, ein höherer Flüssigkeitsdruck ein, ohne dass in Folge des Durchflussbegrenzers 43 ein größerer Volumenstrom aus der Pumpe austreten kann, führt dies dazu, dass die Ventilmembran 33, die in dem Randbereich zwischen dem Mittelteil 3 und dem Oberteil 4 geklemmt ist, sich im zentralen Bereich in Richtung der die Anschlagfunktion aufweisenden Stellschraube 36 verformt, und zwar entgegen der Kraft der Druckfeder 35. Gelangt die Ventilmembran 34 mit ihrem Abschnitt 40 gegen den Vorsprung 55 der Stellschraube 36, der dem Abschnitt 40 zugewandt ist, kontaktiert der Abschnitt 40 dort die Stellschraube 36, womit, da sich die Ventilmembran 33 nicht weiter nach oben in Richtung der Oberschale bewegen kann, der Abschnitt 40 gegen den Vorsprung 54 des Ventilkerns 34 gedrückt wird und damit den Durchfluss durch den Kanal 39 verschließt. Beim Abfließen der Flüssigkeit durch den Durchflussbegrenzer 53 baut sich der Druck in der Kammer 43 ab, sodass die Membran, durchaus auf Grund deren eigenen Elastizität, wieder in Richtung ihrer Ausgangsstellung gemäß Figur 13 zurückbewegt, damit der Abschnitt 40 außer Kontakt mit der Stellschraube 36 gelangt und der Durchflussspalt zwischen dem Vorsprung 54 und dem Abschnitt 40 wieder freigegeben wird. In Abhängigkeit vom Druck, der in dem Ballon 16 herrscht, kann dieser Zustand erst bei Erreichen der Ausgangsposition der Ventilmembran 33, wie sie in Figur 13 dargestellt ist, oder durchaus schon früher, somit bei noch ausgelenkter Ventilmembran 33, eintreten. Die Stellschraube 36 dient der Veränderung des Öffnungs-Schließ-Verhaltens des Ventils 32. Je weiter die Schraube den Stellweg der Ventilmembran freigibt, desto größer ist der Sekundärdruck im Ventil. Grundsätzlich ist es nicht erforderlich, die Druckfeder 35 vorzusehen. Sie ist dann von Vorteil, wenn größere Drücke mit der Pumpe 1 beherrscht werden sollen und demzufolge das elastische Rückstellverhalten der Ventilmembran 33 nicht ausreicht, um sie in die Ausgangsstellung gemäß Figur 13 zu überführen.

Mit dem Ventil 32 wird somit der Flüssigkeits-Volumenstrom in Abhängigkeit vom anstehenden Druck im Ballon 16 begrenzt und über den Durchflussbegrenzer 53 der Flüssigkeits-Volumenstrom weitgehend konstant gehalten. Grundsätzlich könnte die Flüssigkeitspumpe so modifiziert werden, dass nur eine Einrichtung zum weitgehenden Konstanthalten des vom elastischen Element ausgegebenen Flüssigkeits-Volumenstroms oder eine Einrichtung zum Begrenzen des vom elastischen Element ausgegebenen Flüssigkeits-Volumenstroms vorgesehen ist.

Vor dem Benutzen der mechanisch betriebenen Flüssigkeitspumpe wird Flüssigkeit durch das Luer-Lock-Ventil 21 zugeführt, womit die Flüssigkeit in den Ballon 16 gelangt und der Füllstand des Ballons sich durch die durchsichtige Haube 29 an Hand der in Querrichtung der Haube angebrachten Markierungen 51 ablesen lässt, die eine Referenz für die Querausdehnung des Ballons in Abhängigkeit von dessen Befüllung sind. Nach dem Befüllen der Pumpe 1 und dem Anschließen der Pumpe an den Patienten über den Schlauch 50, wird, unter elastischer Vorspannung des aufgeweiteten Ballons 16, Flüssigkeit über das Ventil 32 aus der Pumpe ausgegeben, und zwar solange, bis der Ballon restlos entleert ist und an dem Kern 11 anliegt.

Durch die besonders einfache Gestaltung der beschriebenen Flüssigkeitspumpe bietet diese vielfältige Möglichkeiten der Verwendung bzw. Anwendung. Der Anwender kann überall, sofort, ohne längere Anlaufzeiten die Pumpe einsetzen. Sie kann vom Anwender tragbar, wie auch statisch benutzt werden, und zwar in allen normalen Lebensbereichen außerhalb, wie innerhalb der Medizin. Die Pumpe ist sterilisiert einsetzbar und gewährleistet einen minimalen Bedienungs-/Handlingaufwand. Die Pumpe ist auf Grund der einfachen Konstruktion der wenigen Bauteile kostengünstig herstellbar. Dies ist Vorraussetzung dafür, dass sie insbesondere in ambulanten und finanzschwachen Märkten eingesetzt werden kann. Das geringe Gewicht der Pumpe ermöglicht den Einsatz im Unfall-, Notarzt-, Lazarett- sowie Katastrophenbereich. Die Funktionselemente der Pumpe sind einzeln oder insgesamt austauschbar. Die Pumpe ist für kurze oder lange Förderzeiten geeignet, beispielsweise bei einem Ballon mit einem Fassungsvermögen von 25 ml für eine Durchflussrate von 2,5 ml pro Stunde, somit einer Laufzeit von 10 Stunden. Selbstverständlich können andere Ballons Verwendung finden, die andere Volumina aufweisen, z. B. 10 ml, 50 ml, 100 ml oder 150 ml. Die Laufzeit kann durchaus wesentlich länger sein, beispielsweise bis 24 Stunden. Obwohl Flowraten von > 1000 ml pro Stunde durchaus denkbar sind, wird als bevorzugtes Anwendungsgebiet eine Flowrate von 0,5 bis 10 ml pro Stunde angesehen.

Gemäß dem Ausführungsbeispiel wird eine spritzgusstechnisch hergestellter Ballon beschrieben, der als Sammelbehälter für die Medikamentenlösung und Druckreservoir dient. Der Ballon weist eine definierte Kontur im Querschnitt und in der Ausdehnung, zum Füllen flacher Gehäuseräume und zur Vermeidung von Druckspitzen auf. Er steht radial und/oder axial unter Vorspannung über einen ein- oder mehrteiligen Kern, zur Erhöhung der Rückstellkräfte. Der Ballon wird einseitig über den Kern ortsfest mittels eines Klemmrings formschlüssig, luftdicht abgeschlossen. Der Ballon ist frei beweglich in axialer und radialer Richtung während der Befüllung und Entleerung, dabei elastisch verformbar und kann sich reibungsfrei innerhalb der Haube bewegen.

Die Pumpe 1 kann zusätzlich mit einem Bolusreservoir versehen sein. In der Figur 1 ist eine solche Bolus-Vorsehung mit der Bezugsziffer 52 veranschaulicht. Bei Bedarf kann die Pumpe insofern umgerüstet werden.

Die Figur 14 veranschaulicht in einem Schaubild das Wirkprinzip der zuvor beschriebenen mechanisch betriebenen Flüssigkeitspumpe mit Angabe der physikalischen Größen. Mit strickpunktierter Linie ist die Kontur der Pumpe verdeutlicht.

## Patentansprüche

1. Mechanisch betriebene Flüssigkeitspumpe (1), insbesondere für medizinische oder ernährungsphysiologische Flüssigkeiten, mit einem Gehäuse (2) und einem in diesem gehaltenen, aufweitbaren elastischen Element (16) zum Speichern und Abgeben der Flüssigkeit, ferner mit einem verschließbaren Zugang (21) für die Flüssigkeit zum elastischen Element (16) und einem Abgang (50) für die Flüssigkeit vom elastischen Element, sowie eine Einrichtung (32, 53) zum weitgehenden Konstanthalten und/oder Begrenzen des von dem elastischen Element (16) ausgegebenen Flüssigkeits-Volumenstroms, **gekennzeichnet durch** folgende Merkmale:
- das elastische Element ist als Ballon (16) ausgebildet, der mit einer Öffnung (17a) versehen ist,
- im Gehäuse (2) ist ein Kern (11) gelagert, der **durch** die Öffnung (17a) des Ballons (16) in diesen eingeführt ist,
- es sind Mittel (20) zum Befestigen und Abdichten des Ballons (16) im Bereich seines die Öffnung (17a) aufweisenden Endes (17) am Kern (11) oder am Gehäuse (2) vorgesehen,
- in Abstand von seiner Befestigung liegt der Ballon (16), bei relativ entspanntem Zustand, am Kern (11) an.

2. Pumpe nach Anspruch 1, **dadurch gekennzeichnet, dass** der Ballon (16) aus Silikon besteht.

3. Pumpe nach Anspruch 1 oder 2, **dadurch gekennzeichnet, dass** der Ballon (16) in einer ersten Erstreckungsrichtung (X) senkrecht zur Längsachse des Kerns (11) relativ dicke Wandungsabschnitte (30) und in einer zweiten Erstreckungsrichtung (Y) senkrecht zur Längsachse des Kerns (11) und senkrecht zur ersten Erstreckungsrichtung (X) dünne Wandungsabschnitte (31) aufweist.

4. Pumpe nach einem der Ansprüche 1 bis 3, **dadurch gekennzeichnet, dass** der Ballon (16), in mit der Flüssigkeit gefülltem Zustand, eine flache Form aufweist.

5. Pumpe nach einem der Ansprüche 1 bis 4, **dadurch gekennzeichnet, dass** mit dem Gehäuse (2) eine Haube (29) verbindbar ist, insbesondere eine im Querschnitt, quer zur Längsachse des Kerns geschnitten, nierenförmig gestaltete Haube verbindbar ist.

6. Pumpe nach Anspruch 5, **dadurch gekennzeichnet, dass** das Breiten-/Höhenverhältnis der Haube (29) relativ groß ist, vorzugsweise 1,5:1 bis 3:1, insbesondere ungefähr 2:1 beträgt.

7. Pumpe nach einem der Ansprüche 1 bis 6, **dadurch gekennzeichnet, dass** der Kern (11) rotationssymmetrisch, insbesondere zylindrisch, gestaltet ist und den Kern ein Kanal (13, 14) durchsetzt, der Bestandteil des Zugangs und des Abgangs bildet.

8. Pumpe nach Anspruch 7, **dadurch gekennzeichnet, dass** der Kanal (13, 14) den Kern (11) in dessen Längsmittelachse und dessen radialer Richtung durchsetzt.

9. Pumpe nach einem der Ansprüche 1 bis 8, **dadurch gekennzeichnet, dass** der Ballon (16), in entspanntem Zustand, eine der Form des Kerns (11) im wesentlichen entsprechende Form und den Abmessungen des Kerns (11) im Wesentlichen entsprechende Abmessungen aufweist, insbesondere im Wesentlichen rotationssymmetrisch ausgebildet ist.

10. Pumpe nach einem der Ansprüche 1 bis 9, **dadurch gekennzeichnet, dass** das Längen-/Durchmesserverhältnis des Kerns 3:1 bis 8:1, insbesondere 5:1 bis 6:1 beträgt.

11. Pumpe nach einem der Ansprüche 1 bis 10, **dadurch gekennzeichnet, dass** der Ballon (16) ein Fassungsvermögen von 10 bis 150 ml aufinreist.

12. Pumpe nach einem der Ansprüche 1 bis 11, **gekennzeichnet durch** einen mittels Spritzguss hergestellten Ballon (16).

13. Pumpe nach einem der Ansprüche 1 bis 12, **dadurch gekennzeichnet, dass** der Ballon (16) vollständig an den Kern (11) anlegbar ist.

14. Pumpe nach einem der Ansprüche 1 bis 13, **dadurch gekennzeichnet, dass** die Mittel zum Befestigen und Abdichten des Ballons (16) als Hülse (20) ausgebildet sind, die bei auf den Kern (11) aufgezogenem Ballon (16) diesem zwischen der Hülse (20) und dem Kern (11) klemmt.
